# EUROPÄISCHE PATENTSCHRIFT

(11) **EP 0 371 312 B1**
(45) Veröffentlichungstag und Bekanntmachung des Hinweises auf die Patenterteilung: **20.07.1994**
(21) Anmeldenummer: 89121067.6
(22) Anmeldetag: 14.11.1989
(51) Int. Cl.: C07D 413/04, C07D 413/12, A61K 31/535

(54) **Benzoxazinderivate**
Benzoxazine derivatives
Dérivés de benzoxazine

(30) Priorität: 26.11.1988 DE 3840011
(43) Veröffentlichungstag der Anmeldung: 06.06.1990
(73) Patentinhaber: MERCK PATENT GmbH, D-64271 Darmstadt (DE)
(72) Erfinder: Baumgarth, Manfred, Dr., D-6100 Darmstadt (DE); Gericke, Rolf, Dr., D-6104 Seeheim (DE); Bergmann, Rolf, Dr., D-6101 Reichelsheim (DE); De Peyer, Jacques, Dr., D-6102 Pfungstadt (DE); Lues, Ingeborg, Dr., D-6100 Darmstadt (DE)

(56) Entgegenhaltungen:
- EP-A- 0 175 363
- JP-A-79 118 120
- CHEMICAL & PHARMACEUTICAL BULLETIN,Band 29, Nr. 12, Dezember 1981, Seiten 3529-3535, Pharmaceutical Society ofJapan; R. TACHIKAWA et al.: "Studies on 1,3-benzoxazines. III. Reaction of imidoyl chlorides of 1,3-benzoxazines with 2-hydroxy- or 2-mercaptopyridine N-oxides: a novel S-N bond formation via electrocyclic rearrangement"
- CHEMICAL ABSTRACTS, Band 98, Nr. 3, 17. Januar 1983, Seite 514, Zusammenfassung Nr. 16704s, Columbus, Ohio, US
- CHEMICAL ABSTRACTS, Band 95, Nr. 13, 28. September 1981, Seite 711,Zusammenfassung Nr. 115572s, Columbus, Ohio, US
- CHEMICAL & PHARMACEUTICAL BULLETIN, Band 28, Nr. 2, Februar 1980, Seiten 465-472; K. WACHI et al.: "Studies on 1,3-benzoxazines. I. Synthesis of primary 2-amino-pyridines via the reaction of imidoyl chlorides of 1,3-benzoxazines with pyridine N-oxides"
- CHEMICAL ABSTRACTS, Band 111, Nr. 8, 21. August 1989, Seite 681,Zusammenfassung Nr. 67818b, Columbus, Ohio, US

## Beschreibung

Die Erfindung betrifft neue Benzoxazinderivate der Formel I 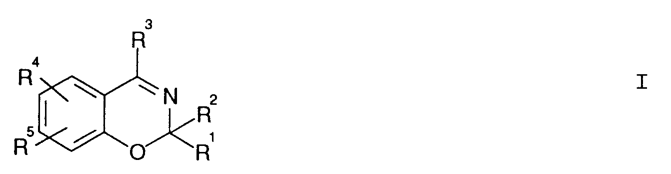
worin
- R¹ und R²: jeweils H oder A, oder zusammen auch Alkylen mit 3-6 C-Atomen,
- R³: einen unsubstituierten oder ein- oder zweifach durch A, F,Cl, Br, I, OH, OA, OAc, SH, NO₂, NH₂, AcNH, HOOC und/oder AOOC substituierten Pyridyl-Z-, wobei 1-Pyridyl-Z ausgenommen ist, Oxo-dihydro-pyridyl-, Oxo-dihydro-pyridazinyl-oxy, Oxo-dihydro-pyridazinyl-thio, Oxo-dihydro-pyridazinyl-amino- oder Oxo-dihydro-pyrrolylrest, wobei die nicht über O, S oder NH an den Benzoxazinring gebundenen Reste auch vollständig oder partiell hydriert sein können,
- R⁴ und R⁵: jeweils H, A, OH, AO, CHO, ACO, ACS, HOOC, AOOC, AO-CS, ACOO, A-CS-O, Hydroxyalkyl mit 1-6 C-Atomen, Mercaptoalkyl mit 1-6 C-Atomen, NO₂, NH₂, NHA, NA₂, CN, F, Cl, Br, I, CF₃, ASO, ASO₂, AO-SO, AO-SO₂, AcNH, AO-CO-NH, H₂NSO, HANSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkyl, Nitroalkyl, Cyanalkyl, A-C(=NOH) oder A-C(=NNH₂),
- A: Alkyl mit 1-6 C-Atomen,
- -alkyl: Alkylen mit 1-6 C-Atomen,
- Ac: Alkanoyl mit 1-8 C-Atomen oder Aroyl mit 7-11 C-Atomen und
- Z: O oder S
bedeuten,
worin jedoch R³ nur dann einen 2-Oxopyrrolidino- oder einen 2-Oxo-piperidino-Rest darstellt und gleichzeitig R⁴ H bedeutet, wenn R⁵ ungleich H, A, AO, F, Cl, Br oder I ist,
sowie deren Salze.

Ähnliche Verbindungen sind aus JP 57,130,979 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze bei guter Verträglichkeit wertvolle pharmakologische Eigenschaften besitzen. So zeigen sie Wirkungen auf das cardiovaskuläre System, wobei in der Regel bei niedrigeren Dosen ein selektiver Angriff am Coronarsystem, bei höheren ein blutdrucksenkender Effekt beobachtet werden kann. Am Coronarsystem treten z. B. Widerstandsabnahme und Flußzunahme auf, wobei der Einfluß auf die Herzfrequenz gering bleibt. Weiterhin zeigen die Verbindungen eine relaxierende Wirkung auf verschiedene glattmuskuläre Organe (Gastrointestinaltrakt, Respirationssystem und Uterus). Die Wirkungen der Verbindungen können mit Hilfe an sich bekannter Methoden ermittelt werden, wie sie z. B. in EP-A-76075, EP-A-168619, EP-A-173848 oder der AU-A-45547/85 (Derwent Farmdoc Nr. 86081769) sowie von K.S. Meesmann et al., Arzneimittelforschung 25 (11), 1975, 1770-1776, angegeben sind. Als Versuchstiere eignen sich z. B. Mäuse, Ratten, Meerschweinchen, Hunde, Katzen, Affen oder Schweine.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe verwendet werden.

In den angegebenen Formeln bedeutet A eine vorzugsweise unverzweigte Alkylgruppe mit 1-6, bevorzugt 1-4, insbesondere 1, 2 oder 3 C-Atomen, im einzelnen vorzugsweise Methyl, ferner bevorzugt Ethyl, Propyl, Isopropyl, Butyl, Isobutyl, weiterhin bevorzugt sek.-Butyl, tert.-Butyl, Pentyl, Isopentyl (3-Methylbutyl), Hexyl oder Isohexyl (4-Methylpentyl).

Falls R¹ und R² zusammen Alkylen bedeuten, so ist die Alkylengruppe vorzugsweise unverzweigt, im einzelnen bevorzugt -(CH₂)ₙ-, wobei n 3, 4, 5 oder 6 bedeutet. Die Gruppe "-alkyl" steht vorzugsweise für -CH₂- oder - CH₂CH₂-.

Ac ist vorzugsweise Alkanoyl mit 1-6, insbesondere 1, 2, 3 oder 4 C-Atomen, im einzelnen bevorzugt Formyl oder Acetyl, weiterhin bevorzugt Propionyl, Butyryl, Isobutyryl, Pentanoyloder Hexanoyl, ferner bevorzugt Benzoyl, o-, m- oder p-Toluyl, 1- oder 2-Naphthoyl.

R¹ und R² sind vorzugsweise jeweils Alkyl, insbesondere jeweils Methyl oder Ethyl, bevorzugt jeweils Methyl.

R³ ist bevorzugt unsubstituiertes 2-Oxo-1,2-dihydro-1-pyridyl (1H-2-Pyridon-1-yl), 3-Hydroxy-6-oxo-1,6-dihydro-pyridazin-1-yl oder 2-Oxo-4-hydroxy-1,2-dihydro-1-pyridyl, ferner bevorzugt unsubstituiertes 6-Oxo-1,6-dihydro-pyridazin-1-yl, 2-Oxo-2,3- oder -2,5-dihydro-pyrrol-1-yl oder 2-Mercapto-pyridyl (= 2-Thioxo-1,2-dihydro-pyridin-1-yl). Falls R³ einen substituierten Pyridon- bzw. Thiopyridonring bedeutet, so ist dieser Ring vorzugsweise einfach in 3-, 4- oder 5-Stellung oder zweifach in 3- und 5-Stellung substituiert. Besonders bevorzugte Substituenten sind OH, NO₂ und NH₂, ferner AOOC, OA, Cl, Br und NHCOCH₃, besonders bevorzugte substituierte Reste R3 im einzelnen 4-, ferner 3-, 5- und 6-Hydroxy-, 3-, 4-, 5- oder 6-Methoxy-, 3-, 4-, 5- oder 6-Acetoxy-, 3-, 5- oder 6-Chlor-, 3- oder 5-Nitro-, 3- oder 5-Amino-, 3- oder 5-Carboxy-, 3- oder 5-Methoxy-carbonyl-, 3- oder 5-Ethoxycarbonyl-, 3- oder 5-Acetamido-, 3,5-Dichlor-, 3,5-Dibrom-, 3-Chlor-5-nitro-, 3-Nitro-5-chlor-, 3-Brom-5-nitro-, 3-Nitro-5-brom-, 3,5-Dinitro-, 3-Chlor-5-amino-, 3-Amino-5-chlor-, 3-Brom-5-amino-, 3-Amino-5-brom-, 3-Chlor-5-acetamido-, 3-Acetamido-5-chlor-, 3-Brom-5-acetamido- und 3-Acetamido-5-brom-2-oxo-1,2-dihydro-1-pyridyl bzw. -2-thioxo-1,2-dihydro-1-pyridyl, 4- oder 5-Hydroxy-6-oxo-1,6-dihydro-pyridazin-1-yl oder 3-, 4- oder 5-Methoxy-6-oxo-1,6-dihydro-pyridazin-1-yl.

Z bedeutet bevorzugt O, ferner S.

R³ kann ferner bevorzugt bedeuten: 2-Oxo-1,2,3,4-tetra-hydro-1-pyridyl, 2-Oxo-1,2,3,6-tetrahydro-1-pyridyl, 2-Oxo-1,2,5,6-tetrahydro-1-pyridyl, 2-Oxo-hexahydro-1-pyridyl (= 2-Piperidinon-1-yl), 2-Oxo-4-hydroxy-1,2,3,4-tetrahydro-1-pyridyl, 2-Oxo-4-hydroxy-1,2,5,6-tetrahydro-1-pyridyl, 2-Oxo-4-hydroxy-hexahydro-1-pyridyl, 6-Oxo-1,2,3,6-tetrahydro-pyridazin-1-yl, 6-Oxo-1,2,5,6-tetra-hydro-pyridazin-1-yl, 6-Oxo-1,4,5,6-tetrahydro-pyridazin-1-yl, 6-Oxo-hexahydro-pyridazin-1-yl, 3-Hydroxy-6-oxo-1,2,3,6-tetrahydro-pyridazin-1-yl, 3-Hydroxy-6-oxo-1,2,5,6-tetrahydro-pyridazin-1-yl, 3-Hydroxy-6-oxo-1,4,5,6-tetrahydro-pyridazin-1-yl, 3-Hydroxy-6-oxo-hexahydro-pyridazin-1-yl, 2-Oxo-tetrahydro-pyrrol-1-yl (= 2-Pyrrolidinon-1-yl).

Falls der Rest R³ eine Z-Gruppe enthält, so ist er bevorzugt 6-Hydroxy-3-pyridazinyl-oxy (= 1,6-Dihydro-6-oxo-3-pyridazinyl-oxy) oder 2-Hydroxy-4-pyridyl-oxy (= 1,2-Dihydro-2-oxo-4-pyridyl-oxy), ferner bevorzugt unsubstituiertes 2-, 3- oder 4-Pyridyl-oxy, 3-, 4- oder 5-Pyridazinyl-oxy; Hydroxy-pyridyl-oxy wie 3-, 4-, 5- oder 6-Hydroxy-2-pyridyl-oxy, 2-, 4- oder 5-Hydroxy-3-pyridyl-oxy, 3-Hydroxy-4-pyridyl-oxy, 2-Hydroxy-5-pyridyl-oxy; Hydroxy-pyridazinyl-oxy wie 4- oder 5-Hydroxy-3-pyridazinyl-oxy, 3-, 5- oder 6-Hydroxy-4-pyridazinyl-oxy; Dihydro-alkyl-oxo-pyridyl-oxy wie 1,2-Dihydro-1-methyl-2-oxo-3-, -4-, -5- oder -6-pyridyl-oxy, 1,2-Dihydro-1-ethyl-2-oxo-3-, -4-, -5- oder -6-pyridyl-oxy; Dihydro-alkyl-oxo-pyridazinyl-oxy wie 1,6-Dihydro-1-methyl-6-oxo-3-, -4- oder -5-pyridazinyl-oxy, 1,6-Dihydro-1-ethyl-6-oxo-3-, -4- oder -5-pyridazinyl-oxy; Alkoxy-pyridyl-oxy wie 3-, 4-, 5- oder 6-Methoxy-2-pyridyl-oxy, 2-, 4- oder 5-Methoxy-3-pyridyl-oxy, 2- oder 3-Methoxy-4-pyridyl-oxy, 2-Methoxy-5-pyridyl-oxy, 2- oder 3-Ethoxy-4-pyridyl-oxy; Alkoxypyridazinyl-oxy wie 4-, 5- oder 6-Methoxy-3-pyridazinyl-oxy, 4-, 5- oder 6-Ethoxypyridazinyl-oxy, 3-, 5- oder 6-Methoxy-4-pyridazinyl-oxy, 3-, 5- oder 6-Ethoxy-4-pyridazinyl-oxy; Amino-pyridyl-oxy wie 3-, 4-, 5- oder 6-Aminopyridyl-oxy, 2-, 4- oder 5-Amino-3-pyridyl-oxy, 2- oder 3-Amino-4-pyridyl-oxy, 2-Amino-5-pyridyl-oxy; Amino-pyridazinyl-oxy wie 4-, 5- oder 6-Amino-3-pyridazinyl-oxy, 3-, 5- oder 6-Amino-4-pyridazinyl-oxy; Mercapto-pyridyl-oxy wie 3-, 4-, 5- oder 6-Mercapto-2-pyridyl-oxy, 2-, 4- oder 5-Mercapto-3-pyridyl-oxy, 2- (= 1,2-Dihydro-2-thioxo-4-pyridyl-oxy) oder 3-Mercapto-4-pyridyl-oxy, 2-Mercapto-5-pyridyl-oxy; Mercapto-pyridazinyl-oxy wie 4-, 5- oder 6-Mercapto-3-pyridazinyl-oxy (= 1,6-Dihydro-6-thioxo-3-pyridazinyl-oxy), 3-, 5- oder 6-Mercapto-4-pyridazinyl-oxy.

Diejenigen Reste R³, die eine einem Ring-N-Atom benachbarte Hydroxy- oder Mercaptogruppe enthalten, können auch in der tautomeren Lactam- oder Thio-lactam-form vorliegen, wie oben in Einzelfällen angegeben.

In R⁴ und R⁵ bedeuten vorzugsweise:
- A:: Methyl, ferner Ethyl;
- AO:: Methoxy, ferner Ethoxy;
- ACO:: Acetyl, ferner Propionyl;
- ACS:: Thioacetyl, ferner Thiopropionyl;
- AOOC:: Methoxycarbonyl, ferner Ethoxycarbonyl;
- AO-CS:: methoxy-thiocarbonyl, ferner Ethoxy-thiocarbonyl;
- ACOO:: Acetoxy, ferner Propionoxy;
- ACSO:: Thio(no)acetoxy, ferner Thio(no)propionoxy;
- Hydroxyalkyl:: Hydroxymethyl oder 1-oder 2-Hydroxyethyl;
- Mercaptoalkyl:: Mercaptomethyl oder 1- oder 2-Mercaptoethyl;
- NHA:: Methylamino, ferner Ethylamino;
- NA₂:: Dimethylamino, ferner Diethylamino;
- ASO:: Methylsulfinyl, ferner Ethylsulfinyl;
- ASO₂:: Methylsulfonyl, ferner Ethylsulfonyl;
- AO-SO:: Methoxy-sulfinyl, ferner Ethoxy-sulfinyl;
- AO-SO₂:: Methoxy-sulfonyl, ferner Ethoxy-sulfonyl;
- Ac-NH:: Acetamido, ferner Formamido, Propionamido oder Benzamido;
- AO-CO-NH:: Methoxycarbonylamino, ferner Ethoxycarbonylamino;
- HANSO:: Methylaminosulfinyl, ferner Ethylaminosulfinyl
- HANSO₂:: Methylaminosulfonyl, ferner Ethylaminosulfonyl;
- A₂NSO₂:: Dimethylminosulfonyl, ferner Diethylaminosulfonyl;
- HANCO:: N-Methylcarbamoyl, ferner N-Ethylcarbamoyl;
- A₂NOC:: N,N-Dimethylcarbamoyl, ferner N,N-Diethylcarbamoyl;
- HANCS:: N-Methyl-thiocarbamoyl, ferner N-Ethylthiocarbamoyl;
- A₂NCS:: N,N-Dimethyl-thiocarbamoyl, ferner N,N-Di-ethyl-thiocarbamoyl;
- ASONH:: Methylsulfinylamino, ferner Ethylsulfinylamino;
- ASO₂NH:: Methylsulfonylamino, ferner Ethylsulfonylamino;
- AOSONH:: Methoxysulfinylamino, ferner Ethoxysulfinylamino;
- AOSO₂NH:: Methoxysulfonylamino, ferner Ethoxysulfonylamino;
- ACO-alkyl:: 2-Oxopropyl, 2-Oxobutyl, 3-Oxobutyl, 3-Oxopentyl;
- Nitroalkyl:: Nitromethyl, 1- oder 2-Nitroethyl;
- Cyanalkyl:: Cyanmethyl, 1- oder 2-Cyanethyl;
- A-C(= NOH):: 1-Oximinoethyl, ferner 1-Oximinopropyl;
- A-C(= NNH₂):: 1-Hydrazonoethyl, ferner 1-Hydrazonopropyl.

Die Reste R⁴ und R⁵ stehen vorzugsweise in 6- und 7-Stellung des Benzoxazinsystems. Sie können jedoch auch in 5-und 6-, 5- und 7-, 5- und 8-, 6- und 8- sowie 7- und 8-Stellung stehen.

Von den Resten R⁴ und R⁵ ist vorzugsweise der eine H, während der andere von H verschieden ist. Dieser andere Rest steht vorzugsweise in 6-Stellung, aber auch in 5-, 7- oder 8-Stellung, und ist vorzugsweise CN, Br oder NO₂, ferner bevorzugt CHO, ACO (insbesondere Acetyl), AOOC (insbesondere Methoxycarbonyl oder Ethoxycarbonyl), ACOO (insbesondere Acetoxy), weiterhin bevorzugt F, Cl, J, CF₃, H₂NCO, H₂NCS oder NH₂.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die nachstehenden Formeln Ia bis Ii ausgedrückt werden, die der Formel I entsprechen und worin die nicht näher bezeichneten Reste die bei der Formel I angegebene Bedeutung haben, worin jedoch
in Ia
- R¹ und R²: jeweils A bedeuten;
in Ib
- R¹ und R²: jeweils CH₃ bedeuten;
in Ic
- R¹ und R²: zusammen Alkylen mit 3-6 C-Atomen bedeuten;
in Id
- R³: 2-Oxo-1,2-dihydro-1-pyridyl, 2-Oxo-4-hydroxy-1,2-dihydro-1-pyridyl oder 3-Hydroxy-6-oxo-1,6-dihydro-1-pyridazinyl bedeutet;
in Ie
- R³: 2-Pyridyloxy, 2-Hydroxy-4-pyridyloxy oder 6-Hydroxy-3-pyridazinyloxy bedeutet;
In If
- R³: 2-Oxo-1,2-dihydro-1-pyridyl bedeutet;
in Ig
- R¹ und R²: jeweils CH₃ und
- R³: 2-Oxo-1,2-dihydro-1-pyridyl, 2-Oxo-4-hydroxy-1,2-dihydro-1-pyridyl oder 3-Hydroxy-6-oxo-1,6-dihydro-1-pyridazinyl bedeuten.
in Ih
- R¹ und R²: jeweils CH₃ und
- R³: 2-Pyridyloxy, 2-Hydroxy-4-pyridyloxy oder 6-hydroxy-3-pyridazinyloxy bedeuten.
in Ii
- R¹ und R²: jeweils CH₃ und
- R³: 2-Oxo-1,2-dihydro-1-pyridyl bedeuten.

Ferner sind bevorzugt Verbindungen der Formeln I sowie Ia bis Ii, worin jeweils zusätzlich
(a)
   - R⁴: von H verschieden ist und
   - R⁵: H bedeutet;
(b)
   - R⁴: von H verschieden ist und in 6-Stellung steht und
   - R⁵: H bedeutet;
(c)
   - R⁴: NO₂, CN, CHO, ACO, HOOC, AOOC, ACOO, F, Cl, Br, J, CF₃, H₂NCO, H₂NCS oder NH₂ und
   - R⁵: H bedeutet;
(d)
   - R⁴: NO₂, CN, CHO, ACO, HOOC, AOOC, ACOO, F, Cl, Br, J, CF₃, H₂NCO, H₂NCS oder NH₂ bedeutet und in 6-Stellung steht und
   - R⁵: H bedeutet;
(e)
   - R⁴: NO₂, CN, CHO, CH₃CO, CH₃OOC, C₂H₅OOC, CH₃COO oder Br und
   - R⁵: H bedeutet;
(f)
   - R⁴: NO₂, CN, CHO, CH₃CO, CH₃OOC, C₂H₅OOC, CH₃COO oder Br bedeutet und in 6-Stellung steht und
   - R⁵: H bedeutet;
(g)
   - R⁴: Br, NO₂ oder CN und
   - R⁵: H bedeutet;
(h)
   - R⁴: Br, NO₂ oder CN bedeutet und in 6-Stellung steht und
   - R⁵: H bedeutet;
(i)
   - R⁴: CN und
   - R⁵: H bedeutet;
(j)
   - R⁴: CN bedeutet und in 6-Stellung steht und
   - R⁵: H bedeutet.

Im übrigen haben vor- und nachstehend die Reste R¹ bis R⁵, Z, A, "-alkyl" und Ac die bei Formel I angegebenen Bedeutungen, wenn nicht ausdrücklich etwas anderes angegeben ist.

Gegenstand der Erfindung ist ferner ein Verfahren zur Herstellung von Benzoxazinderivaten der Formel I, dadurch gekennzeichnet, daß man ein Benzoxazin der Formel II 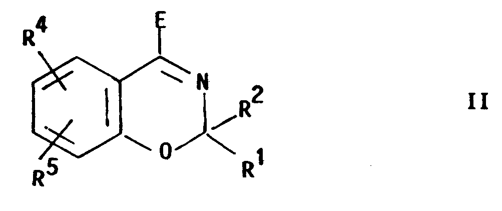
worin
- E: Cl, Br, J oder eine reaktionsfähig veresterte OH-Gruppe bedeutet und
- R¹, R², R⁴ und R⁵: die bei Formel I angegebenen Bedeutungen haben,
mit einer Verbindung der Formel III

R³-H III

worin R³ die bei Formel I angegebene Bedeutung hat,
oder mit einem ihrer reaktionsfähigen Derivate umsetzt und/oder daß man in einer Verbindung der Formel I einen oder mehrere der Reste R³, R⁴ und/oder R⁵ in andere Reste R³, R⁴ und/oder R⁵ umwandelt und/oder daß man eine basische Verbindung der Formel I durch Behandeln mit einer Säure in eines ihrer Säureadditionssalze umwandelt.

Die Verbindungen der Formel I werden im übrigen nach an sich bekannten Methoden hergestellt, wie sie in der Literatur (z. B. in den Standardwerken wie Houben-Weyl, Methoden der organischen Chemie, Georg-Thieme-Verlag, Stuttgart; Organic Reactions, John Wiley & Sons, Inc., New York; sowie in den oben angegebenen Patentanmeldungen) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Vorzugsweise werden die Verbindungen der Formel I durch Reaktion von Verbindungen der Formel II mit Verbindungen der Formel III hergestellt, zweckmäßig in Gegenwart eines inerten Lösungsmittels bei Temperaturen zwischen etwa 0 und 150 °. Dabei können, abhängig von den Reaktionsbedingungen und der Konstitution der Ausgangsstoffe, bei Verwendung von Ausgangsstoffen der Formel III, die eine -CO-NH-Gruppe enthalten (z.B. 1H-2-Pyridon), mit dem Benzoxazinring über das N-Atom [z.B. 4-(2-Oxo-1,2-dihydro-pyridyl)-2H-1,3-benzoxazine] oder über eine O-Brücke [z.B. 4-(2-Pyridyl-oxy)-2H-1,3-benzoxazine] verbundene Verbindungen der Formel I nebeneinander entstehen.

Ausgangsstoffe der Formel II mit E = Cl sind bevorzugt. Sie sind z.B. erhältlich durch Kondensation von Salicylsäureamiden, die gegebenenfalls durch die Reste R⁴ und R⁵ substituiert sind, mit Aldehyden oder Ketonen der Formel R¹-CO-R² zu den entsprechenden 2-R¹-2-R²-2H-1,3-Benzoxazin-4-onen und anschließende Umsetzung mit POCl₃/PCl₅.

In Verbindungen der Formel II, kommen als "reaktionsfähig veresterte OH-Gruppen" insbesondere Alkylsulfonyloxy mit 1-6 C-Atomen (z.B. Methansulfonyloxy) und Arylsulfonyloxy mit 6-10 C-Atomen (z.B. Benzolsulfonyloxy, p-Toluolsulfonyloxy, 1- oder 2-Naphthalinsulfonyloxy) in Betracht.

Die Ausgangsstoffe der Formel III sind in der Regel bekannt.

Als reaktionsfähige Derivate von III eignen sich die entsprechenden Salze, z. B. die Na- oder K-Salze, die auch in situ entstehen können.

Bei der Reaktion von II mit III ist es zweckmäßig, in Gegenwart einer Base zu arbeiten. Als Basen eignen sich z. B. Alkalimetall- oder Erdalkalimetall-hydroxide, -carbonate, -alkoholate, -hydride oder auch -amide wie NaOH, KOH, Ca(OH)₂, Na₂CO₃, K₂CO₃, Na- oder K-methylat, -ethylat oder -tert.-butylat, NaH, KH, CaH₂, NaNH₂, KNH₂, ferner organische Basen wie Triethylamin oder Pyridin, die auch im Überschuß angewendet werden und dann gleichzeitig als Lösungsmittel dienen können.

Als inerte Lösungmittel eignen sich insbesondere Alkohole wie Methanol, Ethanol, Isopropanol, n-Butanol oder tert.-Butanol; Ether wie Diethylether, Diisopropylether, Tetrahydrofuran oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Nitrile wie Acetonitril; Nitroverbindungen wie Nitromethan oder Nitrobenzol; Ester wie Ethylacetat; Amide wie Dimethylformamid (DMF), Dimethylacetamid oder Phosphorsäurehexamethyltriamid; Sulfoxide wie Dimethylsulfoxid (DMSO); chlorierte Kohlenwasserstoffe wie Dichlormethan, Chloroform, Trichlorethylen, 1,2-Dichlorethan oder Kohlenstofftetrachlorid; Kohlenwasserstoffe wie Benzol, Toluol oder Xylol. Weiterhin eignen sich Gemische dieser Lösungsmittel untereinander.

Weiterhin kann man in einer Verbindung der Formel I einen oder mehrere der Reste R³, R⁴ und/oder R⁵ in andere Reste R³, R⁴ und/oder R⁵ umwandeln.

Beispielsweise ist es möglich, daß man ein H-Atom mittels einer Halogenierung durch ein Halogenatom oder mittels einer Nitrierung durch eine Nitrogruppe ersetzt und/oder eine Nitrogruppe zu einer Aminogruppe reduziert und/oder eine Amino- oder Hydroxygruppe alkyliert oder acyliert und/oder eine Cyangruppe (z.B. mit HCl in Wasser/Methanol bei 20-100°) in eine Carboxylgruppe oder (z. B. mit Raney-Nickel in Wasser/Essigsäure/Pyridin in Gegenwart von Natriumphosphat) in eine Formylgruppe oder (z. B. mit KOH in tert.-Butanol) in eine Carbamoylgruppe oder (z. B. mit H₂S in Pyridin/Triethylamin) in eine Thiocarbamoylgruppe umwandelt und/oder eine -CO-NH-Gruppe (z. B. mit P₂S₅ oder mit Lawesson-Reagenz in Toluol) in eine -CS-NH- bzw. eine -C(SH)=N-Gruppe umwandelt.

Eine Nitrierung gelingt unter üblichen Bedingungen, z. B. mit einem Gemisch aus konzentrierter HNO₃ und konzentrierter H₂SO₄ bei Temperaturen zwischen 0 und 30 °. Falls mindestens einer der Substituenten R⁴ und R⁵ eine elektronegative Gruppe wie CN oder NO₂ bedeutet, erfolgt die Nitrierung überwiegend am Rest R³; andernfalls erhält man in der Regel Gemische, bei denen die Nitrogruppen am Rest R³ oder am Benzolring stehen können.

Analoges gilt für die Halogenierung, die z. B. mit elementarem Chlor oder Brom in einem der üblichen inerten Lösungsmittel bei Temperaturen zwischen etwa 0 und 30 ° durchgeführt werden kann.

Eine primäre oder sekundäre Aminogruppe und/oder eine OH-Gruppe kann durch Behandeln mit alkylierenden Mitteln in die entsprechende sekundäre oder tertiäre Aminogruppe und/oder Alkoxygruppe umgewandelt werden. Als alkylierende Mittel eignen sich z. B. Verbindungen der Formeln A-Cl, A-Br oder A-J oder entsprechende Schwefelsäure- oder Sulfonsäureester wie Methylchlorid, -bromid, -jodid, Dimethylsulfat, Methyl-p-toluolsulfonat. Ferner kann man z. B. eine oder zwei Methylgruppen mit Formaldehyd in Gegenwart von Ameisensäure einführen. Die Alkylierung wird zweckmäßig in Gegenwart oder Abwesenheit eines der genannten inerten Lösungsmittel, z. B. DMF, bei Temperaturen zwischen etwa 0 ° und etwa 120 ° vorgenommen, wobei auch ein Katalysator zugegen sein kann, vorzugsweise eine Base wie Kalium-tert.-butylat oder NaH.

Als acylierende Mittel zur Acylierung von Amino- oder Hydroxygruppen eignen sich zweckmäßig die Halogenide (z. B. Chloride oder Bromide) oder Anhydride von Carbonsäuren der Formel Ac-OH, z. B Acetanhydrid, Propionylchlorid, Isobutyrylbromid, Ameisensäure/Essigsäureanhydrid, Benzoylchlorid. Der Zusatz einer Base wie Pyridin oder Triethylamin bei der Acylierung ist möglich. Man acyliert zweckmäßig in Gegenwart oder Abwesenheit eines inerten Lösungsmittels, z. B. eines Kohlenwasserstoffs wie Toluol, eines Nitrils wie Acetonitril, eines Amids wie DMF oder eines Überschusses einer tertiären Base wie Pyridin oder Triethylamin bei Temperaturen zwischen etwa 0 ° und etwa 160 °, vorzugsweise zwischen 20 ° und 120 °. Eine Formylierung gelingt auch mit Ameisensäure in Gegenwart von Pyridin.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z. B. Pikrate, können zur Aufreinigung der Verbindungen der Formel I verwendet werden.

Die Verbindungen der Formel I können ein oder mehrere chirale Zentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr chirale Zentren auf, dann können sie bei der Synthese als Gemische von Racematen anfallen, aus denen man die einzelnen Racemate, beispielsweise durch Umkristallisieren aus inerten Lösungsmitteln, in reiner Form isolieren kann. Erhaltene Racemate können, falls erwünscht, nach an sich bekannten Methoden mechanisch oder chemisch in ihre Enantiomeren getrennt werden. So können aus dem Racemat durch Umsetzung mit einem optisch aktiven Trennmittel Diastereomere gebildet werden. Als Trennmittel für basische Verbindungen der Formel I eignen sich z.B. optisch aktive Säuren, wie die D- und L-Formen von Weinsäure, Dibenzoylweinsäure, Diacetylweinsäure, Camphersulfonsäuren, Mandelsäure, Äpfelsäure oder Milchsäure. Die verschiedenen Formen der Diastereomeren können in an sich bekannter Weise, z.B. durch fraktionierte Kristallisation, getrennt, und die Enantiomeren der Formel I können in an sich bekannter Weise aus den Diastereomeren in Freiheit gesetzt werden. Enantiomerentrennungen gelingen ferner durch Chromatographie an optisch-aktiven Trägermaterialien.

Die Verbindungen der Formel I und ihre physiologisch unbedenklichen Salze können zur Herstellung pharmazeutischer Zübereitungen verwendet werden, insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human-oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes, Pasten, Lotionen, Gele, Sprays, Schäume, Aerosole, Lösungen (z.B. Lösungen in Alkoholen wie Ethanol oder Isopropanol, Acetonitril, DMF, Dimethylacetamid, 1,2-Propandiol oder deren Gemischen untereinander und/oder mit Wasser) oder Puder. Die neuen Verbindungen können auch lyophilisiert und die erhaltenen Lyophilisate z.B. zur Herstellung von Injektionspräparaten verwendet werden. Insbesondere für die topische Anwendung kommen auch liposomale Zubereitungen in Betracht. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können an Menschen oder Tiere, insbesondere Säugetiere wie Affen, Hunde, Katzen, Ratten oder Mäuse verabreicht und bei der therapeutischen Behandlung des menschlichen oder tierischen Körpers sowie bei der Bekämpfung von Krankheiten verwendet werden, insbesondere bei der Therapie und/oder Prophylaxe von Störungen des cardiovasculären Systems, insbesondere dekompensierter Herzinsuffizienz, Angina pectoris, Arrhythmie, peripheren oder cerebralen Gefäßerkrankungen, sowie Krankheitszuständen, die mit Bluthochdruck verbunden sind, ferner von Erkrankungen, die mit Veränderungen der nicht-vaskulären Muskulatur verbunden sind, z.B. Asthma, Inkontinenz der Harnblase.

Dabei werden die erfindungsgemäßen Substanzen in der Regel in Analogie zu bekannten Antianginosa bzw. Blutdrucksenkern, z. B. Nicorandil oder Cromakalim verabreicht, vorzugsweise in Dosierungen zwischen etwa 0,01 und 5 mg, insbesondere zwischen 0,02 und 0,5 mg pro Dosierungseinheit. Die tägliche Dosierung liegt vorzugsweise zwischen etwa 0,0001 und 0,1, insbesondere zwischen 0,0003 und 0,01 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Die Verbindungen der Formel I und ihre Salze eignen sich, insbesondere bei topischer Anwendung, ferner zur Behandlung der Alopezie einschließlich der androgenen Alopezie und der Alopecia areata. Hierfür werden insbesondere pharmazeutische Zubereitungen verwendet, die zur topischen Behandlung der Kopfhaut geeignet und die oben genannt sind. Sie enthalten etwa 0,005 bis 10, bevorzugt 0,5 bis 3 Gew.% mindestens einer Verbindung der Formel I und/oder mindestens eines ihrer Salze. Im übrigen können diese Verbindungen gegen Alopezie in Analogie zu den Angaben in der WO 88/00822 verwendet werden.

In den nachfolgenden Beispielen bedeutet "übliche Aufarbeitung":
Man gibt, falls erforderlich, Wasser hinzu, extrahiert mit einem organischen Lösungsmittel wie Ethylacetat, trennt ab, trocknet die organische Phase über Natriumsulfat, filtriert, dampft ein und reinigt durch Chromatographie und/oder Kristallisation.

Vor- und nachstehend sind alle Temperaturen in °C angegeben.

### Beispiel 1

Zu einer Anschlämmung von 741 mg NaH (80%ig) in 40 ml trockenem DMF gibt man unter Einleiten von N₂ und Rühren 1,9 g 1H-2-Pyridon. Nach 0,5 Std. Rühren setzt man 2,2 g 2,2-Dimethyl-4-chlor-6-cyan-2H-1,3-benzoxazin ("IIa"; F. 127-129°; erhältlich durch Kondensation von 5-Cyan-2-hydroxy-benzamid mit Aceton zu 2,2-Dimethyl-6-cyan-3,4-dihydro-2H-1,3-benzoxazin-4-on (F. 213-216°) und anschließende Umsetzung mit POCl₃/PCl₅) hinzu und rührt weitere 72 Std. bei 50°. Danach versetzt man mit gesättigter NaCl-Lösung, extrahiert mit Ethylacetat, wäscht mit NaCl-Lösung, trocknet über Natriumsulfat, dampft ein und chromatographiert an Kieselgel (Ethylacetat/Petrolether). Man erhält als erste Fraktion 2,2-Dimethyl-4-(2-pyridyl-oxy)-6-cyan-2H-1,3-benzoxazin ("A"; F. 115-116°), als zweite Fraktion 2,2-Dimethyl-4-(2-oxo-1,2-dihydropyridyl)-6-cyan-2H-1,3-benzoxazin ("B"; F. 158-159°).

Analog erhält man mit 2,4-Dihydroxypyridin (= 4-Hydroxy-1H-2-pyridon) das 2,2-Dimethyl-4-(2-oxo-4-hydroxy-1,2-dihydro-1-pyridyl)-6-cyan-2H-1,3-benzoxazin und das 2,2-Dimethyl-4-(2-hydroxy-4-pyridyloxy)-6-cyan-2H-1,3-benzoxazin, F. 275-278°.

Analog erhält man mit 3,6-Dihydroxypyridazin (= 3-Hydroxy-6-oxo-1,6-dihydro-pyridazin) das 2,2-Dimethyl-4-(3-hydroxy-6-oxo-1,6-dihydro-1-pyridazinyl)-6-cyan-2H-1,3-benzoxazin und das 2,2-Dimethyl-4-(6-hydroxy-3-pyridazinyl-oxy)-6-cyan-2H-1,3-benzoxazin, F. 189-190°.

Analog erhält man:
2,2-Dimethyl-4-(2-oxo-1,2-dihydro-1-pyridyl)-2H-1,3-benzoxazin, F. 144-145°
2,2-Dimethyl-4-(2-pyridyl-oxy)-2H-1,3-benzoxazin
2,2-Dimethyl-4-(2-thioxo-1,2-dihydro-1-pyridyl)-6-cyan-2H-1,3-benzoxazin
2,2-Dimethyl-4-(2-pyridyl-thio)-6-cyan-2H-1,3-benzoxazin
2,2-Dimethyl-4-(2-oxo-3-chlor-1,2-dihydro-1-pyridyl)-6-cyan-2H-1,3-benzoxazin
2,2-Dimethyl-4-(3-chlor-2-pyridyl-oxy)-6-cyan-2H-1,3-benzoxazin
2,2-Dimethyl-4-(2-oxo-5-chlor-1,2-dihydro-1-pyridyl)-6-cyan-2H-1,3-benzoxazin
2,2-Dimethyl-4-(5-chlor-2-pyridyl-oxy)-6-cyan-2H-1,3-benzoxazin
2,2-Dimethyl-4-(2-oxo-6-chlor-1,2-dihydro-1-pyridyl)-6-cyan-2H-1,3-benzoxazin
2,2-Dimethyl-4-(6-chlor-2-pyridyl-oxy)-6-cyan-2H-1,3-benzoxazin
2,2-Dimethyl-4-(2-oxo-3-hydroxy-1,2-dihydro-1-pyridyl)-6-2H-1,3-benzoxazin
2,2-Dimethyl-4-(3-hydroxy-2-pyridyl-oxy)-6-cyan-2H-1,3-benzoxazin
2,2-Dimethyl-4-(2-oxo-5-hydroxy-1,2-dihydro-1-pyridyl)-6-cyan-2H-1,3-benzoxazin
2,2-Dimethyl-4-(5-hydroxy-2-pyridyl-oxy)-6-cyan-2H-1,3-benzoxazin
2,2-Dimethyl-4-(2-oxo-3-methoxy-1,2-dihydro-1-pyridyl)-6-cyan-2H-1,3-benzoxazin
2,2-Dimethyl-4-(3-methoxy-2-pyridyl-oxy)-6-cyan-2-H-1,3-benzoxazin
2,2-Dimethyl-4-(2-oxo-3-acetoxy-1,2-dihydro-1-pyridyl)-6-cyan-2H-1,3-benzoxazin
2,2-Dimethyl-4-(3-acetoxy-2-pyridyl-oxy)-6-cyan-2H-1,3-benzoxazin
2,2-Dimethyl-4-(2-oxo-3-nitro-1,2-dihydro-1-pyridyl)-6-cyan-2H-1,3-benzoxazin
2,2-Dimethyl-4-(3-nitro-2-pyridyl-oxy)-6-cyan-2H-1,3-benzoxazin
2,2-Dimethyl-4-(2-oxo-5-nitro-1,2-dihydro-1-pyridyl)-6-cyan-2H-1,3-benzoxazin
2,2-Dimethyl-4-(5-nitro-2-pyridyl-oxy)-6-cyan-2H-1,3-benzoxazin
2,2-Dimethyl-4-(2-oxo-3-amino-1,2-dihydro-1-pyridyl)-6-cyan-2H-1,3-benzoxazin
2,2-Dimethyl-4-(3-amino-2-pyridyl-oxy)-6-cyan-2H-1,3-benzoxazin
2,2-Dimethyl-4-(2-oxo-5-amino-1,2-dihydro-1-pyridyl)-6-cyan-2H-1,3-benzoxazin
2,2-Dimethyl-4-(5-amino-2-pyridyl-oxy)-6-cyan-2H-1,3-benzoxazin
2,2-Dimethyl-4-(2-oxo-3-acetamido-1,2-dihydro-1-pyridyl)-6-cyan-2H-1,3-benzoxazin
2,2-Dimethyl-4-(3-acetamido-2-pyridyl-oxy)-6-cyan-2H-1,3-benzoxazin
2,2-Dimethyl-4-(2-oxo-5-acetamido-1,2-dihydro-1-pyridyl)-6-cyan-2H-1,3-benzoxazin
2,2-Dimethyl-4-(5-acetamido-2-pyridyl-oxy)-6-cyan-2H-1,3-benzoxazin
2,2-Dimethyl-4-(2-oxo-3-carboxy-1,2-dihydro-1-pyridyl)-6-cyan-2H-1,3-benzoxazin
2,2-Dimethyl-4-(3-carboxy-2-pyridyl-oxy)-6-cyan-2H-1,3-benzoxazin
2,2-Dimethyl-4-(2-oxo-3,5-dichlor-1,2-dihydro-1-pyridyl)-6-cyan-2H-1,3-benzoxazin
2,2-Dimethyl-4-(3,5-dichlor-2-pyridyl-oxy)-6-cyan-2H-1,3-benzoxazin
2,2-Dimethyl-4-(2-oxo-3,5-dibrom-1,2-dihydro-1-pyridyl)-6-cyan-2H-1,3-benzoxazin
2,2-Dimethyl-4-(3,4-dibrom-2-pyridyl-oxy)-6-cyan-2H-1,3-benzoxazin
2,2-Dimethyl-4-(2-oxo-3-chlor-5-nitro-1,2-dihydro-1-pyridyl)-6-cyan-2H-1,3-benzoxazin
2,2-Dimethyl-4-(3-chlor-5-nitro-2-pyridyl-oxy)-6-cyan-2H-1,3-benzoxazin
2,2-Dimethyl-4-(2-oxo-3-nitro-5-chlor-1,2-dihydro-1-pyridyl)-6-cyan-2H-1,3-benzoxazin
2,2-Dimethyl-4-(3-nitro-5-chlor-2-pyridyl-oxy)-6-cyan-2H-1,3-benzoxazin
2,2-Dimethyl-4-(2-oxo-3-brom-5-nitro-1,2-dihydro-1-pyridyl)-6-cyan-2H-1,3-benzoxazin
2,2-Dimethyl-4-(3-brom-5-nitro-2-pyridyl-oxy)-6-cyan-2H-1,3-benzoxazin
2,2-Dimethyl-4-(2-oxo-3-nitro-5-brom-1,2-dihydro-1-pyridyl)-6-cyan-2H-1,3-benzoxazin
2,2-Dimethyl-4-(3-nitro-5-brom-2-pyridyl-oxy)-6-cyan-2H-1,3-benzoxazin
2,2-Dimethyl-4-(2-oxo-3,5-dinitro-1,2-dihydro-1-pyridyl)-6-cyan-2H-1,3-benzoxazin
2,2-Dimethyl-4-(3,5-dinitro-2-pyridyl-oxy)-6-cyan-2H-1,3-benzoxazin
2,2-Dimethyl-4-(2-oxo-3-chlor-5-amino-1,2-dihydro-1-pyridyl)-6-cyan-2H-1,3-benzoxazin
2,2-Dimethyl-4-(3-chlor-5-amino-2-pyridyl-oxy)-6-cyan-2H-1,3-benzoxazin
2,2-Dimethyl-4-(2-oxo-3-amino-5-chlor-1,2-dihydro-1-pyridyl)-6-cyan-2H-1,3-benzoxazin
2,2-Dimethyl-4-(3-amino-5-chlor-2-pyridyl-oxy)-6-cyan-2H-1,3-benzoxazin
2,2-Dimethyl-4-(2-oxo-3-brom-5-amino-1,2-dihydro-1-pyridyl)-6-cyan-2H-1,3-benzoxazin
2,2-Dimethyl-4-(3-brom-5-amino-2-pyridyl-oxy)-6-cyan-2H-1,3-benzoxazin
2,2-Dimethyl-4-(2-oxo-3-amino-5-brom-1,2-dihydro-1-pyridyl)-6-cyan-2H-1,3-benzoxazin
2,2-Dimethyl-4-(3-amino-5-brom-2-pyridyl-oxy)-6-cyan-2H-1,3-benzoxazin
2,2-Dimethyl-4-(2-oxo-3-chlor-5-acetamido-1,2-dihydro-1-pyridyl)-6-cyan-2H-1,3-benzoxazin
2,2-Dimethyl-4-(3-chlor-5-acetamido-2-pyridyl-oxy)-6-cyan-2H-1,3-benzoxazin
2,2-Dimethyl-4-(2-oxo-3-acetamido-5-chlor-1,2-dihydro-1-pyridyl)-6-cyan-2H-1,3-benzoxazin
2,2-Dimethyl-4-(3-acetamido-5-chlor-2-pyridyl-oxy)-6-cyan-2H-1,3-benzoxazin
2,2-Dimethyl-4-(2-oxo-3-brom-5-acetamido-1,2-dihydro-1-pyridyl)-6-cyan-2H-1,3-benzoxazin
2,2-Dimethyl-4-(3-brom-5-acetamido-2-pyridyl-oxy)-6-cyan-2H-1,3-benzoxazin
2,2-Dimethyl-4-(2-oxo-3-acetamido-5-brom-1,2-dihydro-1-pyridyl)-6-cyan-2H-1,3-benzoxazin
2,2-Dimethyl-4-(3-acetamido-5-brom-2-pyridyl-oxy)-6-cyan-2H-1,3-benzoxazin
2,2-Dimethyl-4-(2-oxo-1,2-dihydro-1-pyridyl)-6-acetyl-2H-1,3-benzoxazin
2,2-Dimethyl-4-(2-pyridyl-oxy)-6-acetyl-2H-1,3-benzoxazin
2,2-Dimethyl-4-(2-oxo-1,2-dihydro-1-pyridyl)-6-methoxycarbonyl-2H-1,3-benzoxazin
2,2-Dimethyl-4-(2-pyridyl-oxy)-6-methoxycarbonyl-2H-1,3-benzoxazin
2,2-Dimethyl-4-(2-oxo-1,2-dihydro-1-pyridyl)-6-ethoxycarbonyl-2H-1,3-benzoxazin
2,2-Dimethyl-4-(2-pyridyl-oxy)-6-ethoxycarbonyl-2H-1,3-benzoxazin
2,2-Dimethyl-4-(2-oxo-1,2-dihydro-1-pyridyl)-6-fluor-2H-1,3-benzoxazin
2,2-Dimethyl-4-(2-pyridyl-oxy)-6-fluor-2H-1,3-benzoxazin
2,2-Dimethyl-4-(2-oxo-1,2-dihydro-1-pyridyl)-6-chlor-2H-benzoxazin, F. 114°
2,2-Dimethyl-4-(2-pyridyl-oxy)-6-chlor-2H-1,3-benzoxazin
2,2-Dimethyl-4-(2-oxo-1,2-dihydro-1-pyridyl)-6-brom-2H-1,3-benzoxazin, F. 134° (über 2,2-Dimethyl-6-brom-3,4-dihydro-2H-1,3-benzoxazin-4-on, F 169-175°, und
2,2-Dimethyl-4-chlor-6-brom-2H-1,3-benzoxazin, Kp. 150-190°/1,3-2,7 mbar, Luftbad, Kugelrohr-Destillation)
2,2-Dimethyl-4-(2-pyridyl-oxy)-6-brom-2H-1,3-benzoxazin
2,2-Dimethyl-4-(2-oxo-1,2-dihydro-1-pyridyl)-6-nitro-2H-1,3-benzoxazin
2,2-Dimethyl-4-(2-pyridyl-oxy)-6-nitro-2H-1,3-benzoxazin
2,2-Dimethyl-4-(2-oxo-1,2-dihydro-1-pyridyl)-6-trifluormethyl-2H-1,3-benzoxazin
2,2-Dimethyl-4-(2-pyridyl-oxy)-6-trifluormethyl-2H-1,3-benzoxazin
2,2-Dimethyl-4-(2-oxo-1,2-dihydro-1-pyridyl)-6-acetamido-2H-1,3-benzoxazin
2,2-Dimethyl-4-(2-pyridyl-oxy)-6-acetamido-2H-1,3-benzoxazin
2,2-Dimethyl-4-(2-oxo-1,2-dihydro-1-pyridyl)-7-cyan-2H-1,3-benzoxazin
2,2-Dimethyl-4-(2-pyridyl-oxy)-7-cyan-2H-1,3-benzoxazin
2,2-Dimethyl-4-(2-oxo-1,2-dihydro-1-pyridyl)-6-acetamido-7-nitro-2H-1,3-benzoxazin
2,2-Dimethyl-4-(2-pyridyl-oxy)-6-acetamido-7-nitro-2H-1,3-benzoxazin
2-Methyl-4-(2-oxo-1,2-dihydro-1-pyridyl)-6-cyan-1,2-2H-1,3-benzoxazin
2-Methyl-4-(2-pyridyl-oxy)-6-cyan-2H-1,3-benzoxazin
2-Methyl-2-ethyl-4-(2-oxo-1,2-dihydro-1-pyridyl)-6-cyan-2H-1,3-benzoxazin
2-Methyl-2-ethyl-4-(2-pyridyl-oxy)-6-cyan-2H-1,3-benzoxazin
2,2-Diethyl-4-(2-oxo-1,2-dihydro-1-pyridyl)-6-cyan-2H-1,3-benzoxazin
2,2-Diethyl-4-(2-pyridyl-oxy)-6-cyan-2H-1,3-benzoxazin
2,2-Tetramethylen-4-(2-oxo-1,2-dihydro-1-pyridyl)-6-cyan-2H-1,3-benzoxazin
2,2-Tetramethylen-4-(2-Pyridyl-oxy)-6-cyan-2H-1,3-benzoxazin
2,2-Pentamethylen-4-(2-oxo-1,2-dihydro-1-pyridyl)-6-cyan-2H-1,3-benzoxazin
2,2-Pentamethylen-4-(2-pyridyl-oxy)-6-cyan-2H-1,3-benz-oxazin
2,2,3-Trimethyl-4-(2-oxo-1,2-dihydro-1-pyridyl)-6-cyan-2H-1,3-benzoxazin
2,2,3-Trimethyl-4-(2-pyridyl-oxy)-6-cyan-2H-1,3-benzoxazin
2,2-Dimethyl-4-(6-oxo-1,6-dihydro-1-pyridazinyl)-6-cyan-2H-1,3-benzoxazin
2,2-Dimethyl-4-(3-pyridazinyl-oxy)-6-cyan-2H-1,3-benzoxazin
2,2-Dimethyl-4-(3-ethoxycarbonyl-6-oxo-1,6-dihydro-1-pyridazinyl)-6-cyan-2H-1,3-benzoxazin
2,2-Dimethyl-4-(6-ethoxycarbonyl-3-pyridazinyl-oxy)-6-cyan-2H-1,3-benzoxazin
2,2-Dimethyl-4-(2-oxo-2,5-dihydro-1-pyrrolyl)-6-cyan-2H-1,3-benzoxazin
2,2-Dimethyl-4-(2-pyrrolyl-oxy)-6-cyan-2H-1,3-benzoxazin
2,2-Dimethyl-4-(2-oxo-4-hydroxy-1,2-dihydro-1-pyridyl)-6-brom-2H-1,3-benzoxazin
2,2-Dimethyl-4-(2-hydroxy-4-pyridyl-oxy)-6-brom-2H-1,3-benzoxazin, F. 171-172°
2,2-Dimethyl-4-(2-oxo-4-hydroxy-1,2-dihydro-1-pyridyl)-6-nitro-2H-1,3-benzoxazin
2,2-Dimethyl-4-(2-hydroxy-4-pyridyl-oxy)-6-nitro-2H-1,3-benzoxazin
2,2-Dimethyl-4-(2-oxo-4-hydroxy-1,2-dihydro-1-pyridyl)-6-methoxycarbonyl-2H-1,3-benzoxazin
2,2-Dimethyl-4-(2-hydroxy-4-pyridyl-oxy)-6-methoxycarbonyl-2H-1,3-benzoxazin
2,2-Dimethyl-4-(3-hydroxy-6-oxo-1,6-dihydro-1-pyridazinyl)-6-brom-2H-1,3-benzoxazin
2,2-Dimethyl-4-(6-hydroxy-3-pyridazinyl-oxy)-6-brom-2H-1,3-benzoxazin, F. 196-198°
2,2-Dimethyl-4-(3-hydroxy-6-oxo-1,6-dihydro-1-pyridazinyl)-6-nitro-2H-1,3-benzoxazin
2,2-Dimethyl-4-(6-hydroxy-3-pyridazinyl-oxy)-6-nitro-2H-1,3-benzoxazin
2,2-Dimethyl-4-(3-hydroxy-6-oxo-1,6-dihydro-1-pyridazinyl)-6-methoxycarbonyl-2H-1,3-benzoxazin
2,2-Dimethyl-4-(6-hydroxy-3-pyridazinyl-oxy)-6-nitro-2H-2,3-benzoxazin.

### Beispiel 2

Ein Gemisch von 22 g IIa, 1,9 g 1H-2-Pyridon, 1 ml Pyridin und 10 ml Ethanol wird 2 Std. auf 100° (im Rohr) erhitzt. Man dampft ein, arbeitet wie üblich auf und erhält bei der chromatographischen Trennung "A" und "B".

### Beispiel 3

Eine Lösung von 2,65 g 2,2-Dimethyl-4-brom-6-cyan-2H-1,3-benzoxazin (erhältlich aus 2,2-Dimethyl-6-cyan-3,4-dihydro-2H-1,3-benzoxazin-4-on und SOBr₂) und 0,95 g 2H-1-Pyridon in 20 ml DMSO wird mit 1,2 g 80%igem NaH versetzt und 3 Tage bei 20° gerührt. Nach üblicher Aufarbeitung erhält man "A" und "B".

### Beispiel 4

Analog Beispiel 2 erhält man aus IIa und 2-Amino-3-hydroxypyridin das 2,2-Dimethyl-4-(3-hydroxy-2-pyridylamino)-6-cyan-2H-1,3-benzoxazin.

### Beispiel 5

Analog Beispiel 1 erhält man aus IIa und Pyrrolidin-2-on das 2,2-Dimethyl-4-(2-oxo-pyrrolidino)-6-cyan-2H-1,3-benzoxazin, F. 186-187°.

Analog erhält man:
2,2-Dimethyl-4-(2-oxo-pyrrolidino)-6-nitro-2H-1,3-benzoxazin
2,2-Dimethyl-4-(2-oxo-piperidino)-6-cyan-2H-1,3-benzoxazin
2,2-Dimethyl-4-(2-oxo-piperidino)-6-nitro-2H-1,3-benzoxazin.

### Beispiel 6

Eine Lösung von 1 g 2,2-Dimethyl-4-(2-oxo-1,2-dihydro-1-pyridyl)-6-nitro-2H-1,3-benzoxazin in 24 ml Methanol wird bei 20° und 1 bar an 0,5 g 5%igem Pd-C bis zum Stillstand hydriert. Man filtriert, dampft ein und erhält nach üblicher Aufarbeitung (mit verdünnter Natronlauge/Dichlormethan) 2,2-Dimethyl-4-(2-oxo-1,2-dihydro-1-pyridyl)-6-amino-2H-1,3-benzoxazin.

### Beispiel 7

Eine Lösung von 1 g 2,2-Dimethyl-4-(2-oxo-1,2-dihydro-1-pyridyl)-6-amino-2H-1,3-benzoxazin in 15 ml Ameisensäure und 1 ml Pyridin wird 16 Std. gekocht und eingedampft. Nach üblicher Aufarbeitung erhält man 2,2-Dimethyl-4-(2-oxo-1,2-dihydro-1-pyridyl)-6-formamido-2H-1,3-benzoxazin.

### Beispiel 8

Ein Gemisch von 1 g 2,2-Dimethyl-4-(2-oxo-1,2-dihydro-1-pyridyl)-6-amino-2H-1,3-benzoxazin, 10 ml Acetanhydrid und 10 ml Pyridin wird 16 Std. bei 20° stehengelassen. Man dampft ein, reinigt chromatographisch und erhält 2,2-Dimethyl-4-(2-oxo-1,2-dihydro-1-pyridyl)-6-acetamido-2H-1,3-benzoxazin.

### Beispiel 9

In eine siedende Lösung von 1 g "B" in 50 ml Methanol und 2 ml Wasser wird 14 Std. unter Rühren HCl eingeleitet. Man kühlt über Nacht auf 0°. Die ausgefallene 2,2-Dimethyl-4-(2-oxo-1,2-dihydro-1-pyridyl)-2H-1,3-benzoxazin-6-carbonsäure wird abfiltriert.

### Beispiel 10

Ein Gemisch von 2,79 g "B", 31 g Na₃PO₄ · 12 H₂O, 28 ml Pyridin, 28 ml Wasser, 67 ml Essigsäure und 25 g Raney-Nickel (wasserfeucht) wird 3 Std. bei 20° gerührt. Nach Filtration arbeitet man wie üblich auf und erhält 2,2-Dimethyl-4-(2-oxo-1,2-dihydro-1-pyridyl)-6-formyl-2H-1,3-benzoxazin.

### Beispiel 11

Man löst 2,79 g "B" in 40 ml tert.-Butanol und versetzt unter Rühren mit 5,6 g gepulvertem KOH. Nach einstündigem Kochen und üblicher Aufarbeitung erhält man 2,2-Dimethyl-4-(2-oxo-1,2-dihydro-1-pyridyl)-6-carbamoyl-2H-1,3-benzoxazin.

### Beispiel 12

In eine Lösung von 2,79 g "B" in einem Gemisch von 20 ml Pyridin und 10 ml Triethylamin leitet man bei 20° 5 Std. H₂S ein, dampft ein, arbeitet wie üblich auf und erhält 2,2-Dimethyl-4-(2-oxo-1,2-dihydro-1-pyridyl)-6-thiocarbamoyl-2H-1,3-benzoxazin.

### Beispiel 13

Ein Gemisch von 2,79 g "B", 8,08 g Lawesson-Reagenz und 50 ml Toluol wird 1 Std. unter N₂ gekocht. Übliche Aufarbeitung gibt 2,2-Dimethyl-4-(2-thioxo-1,2-dihydro-1-pyridyl)-6-cyan-2H-1,3-benzoxazin.

Analog erhält man
2,2-Dimethyl-4-(2-thioxo-1,2-dihydro-1-pyridyl)-6-brom-2H-1,3-benzoxazin
2,2-Dimethyl-4-(2-thioxo-1,2-dihydro-1-pyridyl)-6-nitro-2H-1,3-benzoxazin.

### Beispiel 14

Ein Gemisch von 295 mg 2,2-Dimethyl-4-(2-oxo-1,2-dihydro-4-pyridyloxy)-6-cyan-2H-1,3-benzoxazin, 20 ml Aceton, 400 mg K₂CO₃ und 0,2 ml Dimethylsulfat wird 2 Std. gekocht. Man filtriert, arbeitet wie üblich auf und erhält 2,2-Dimethyl-4-(1-methyl-2-oxo-1,2-dihydro-4-pyridyloxy)-6-cyan-2H-1,3-benzoxazin.

### Beispiel 15

Ein Gemisch von 296 mg 2,2-Dimethyl-4-(6-hydroxy-3-pyridazinyloxy)-6-cyan-2H-1,3-benzoxazin, 1 g K₂CO₃, 0,65 ml Dimethylsulfat und 16 ml DMF wird 3 Std. gekocht und wie üblich aufgearbeitet. Man erhält 2,2-Dimethyl-4-(6-methoxy-3-pyridazinyloxy)-6-cyan-2H-1,3-benzoxazin.

### Beispiel 16

Analog Beispiel 1 erhält man aus 2,2-Dimethyl-4-chlor-6-methoxy-2H-1,3-benzoxazin und 1H-2-Pyridon das 2,2-Dimethyl-4-(2-oxo-1,2-dihydro-pyridyl)-6-methoxy-2H-1,3-benzoxazin, F. 104°.

Analog erhält man aus IIa und 3-Mercapto-6-hydroxy-pyridazin das 2,2-Dimethyl-4-(6-hydroxy-3-pyridazinyl-thio)-6-cyan-2H-1,3-benzoxazin.

Analog erhält man mit 2,2-Dimethyl-4-chlor-6-brom-2H-1,3-benzoxazin das 2,2-Dimethyl-4-(6-hydroxy-3-pyridazinyl-thio)-6-brom-2H-1,3-benzoxazin.

### Beispiel 17

Eine Lösung von Diazomethan in Ether wird bei 20° tropfenweise bis zur bleibenden Gelbfärbung zugegeben zu einer Lösung von 1 g 2,2-Dimethyl-4-(6-hydroxy-3-pyridazinyloxy)-6-cyan-2H-1,3-benzoxazin. Man dampft ein und erhält 2,2-Dimethyl-4-(1,6-dihydro-1-methyl-6-oxo-3-pyridazinyloxy)-6-cyan-2H-benzoxazin.

Analog erhält man aus der entsprechenden Bromverbindung das 2,2-Dimethyl-4-(1,6-dihydro-1-methyl-6-oxo-3-pyridazinyl-oxy)-6-brom-2H-1,3-benzoxazin.

Die nachstehenden Beispiele betreffen pharmazeutische Zübereitungen, die Verbindungen der Formel I oder ihre physiologisch unbedenklichen Salze enthalten:

### Beispiel A Tabletten

Ein Gemisch von 1 kg "B", 80 kg Lactose, 24 kg Kartoffelstärke, 4 kg Talk und 2 kg Magnesiumstearat wird in üblicher Weise zu Tabletten gepreßt, derart, daß jede Tablette 1 mg Wirkstoff enthält.

### Beispiel B Dragees

Analog Beispiel A werden Tabletten gepreßt, die anschließend in üblicher Weise mit einem Überzug aus Saccharose, Kartoffelstärke, Talk, Tragant und Farbstoff überzogen werden.

### Beispiel C Kapseln

Man füllt 1 kg 2,2-Dimethyl-4-(6-hydroxy-3-pyridazinyloxy)-6-cyan-2H-1,3-benzoxazin in üblicher Weise in Hartgelatinekapseln, so daß jede Kapsel 0,5 mg Wirkstoff enthält.

### Beispiel D Ampullen

Eine Lösung von 50 g "B" in 70 l 1,2-Propandiol wird mit zweifach destilliertem Wasser auf 100 l aufgefüllt, steril filtriert, in Ampullen abgefüllt und steril verschlossen. Jede Ampulle enthält 0,5 mg Wirkstoff.

Analog sind Tabletten, Dragees, Kapseln oder Ampullen erhältlich, die einen oder mehrere der übrigen Wirkstoffe der Formel I und/oder ihrer physiologisch unbedenklichen Salze enthalten.

## Patentansprüche (Patentansprüche für folgende(n) Vertragsstaat(en): AT, BE, CH, DE, FR, GB, GR, IT, LI, LU, NL, SE)

1. Benzoxazinderivate der Formel I 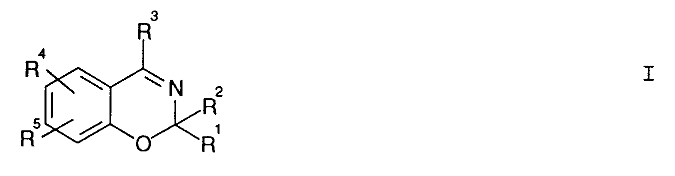 worin
R¹ und R² jeweils H oder A, oder zusammen auch Alkylen mit 3-6 C-Atomen,
R³ einen unsubstituierten oder ein- oder zweifach durch A, F, Cl, Br, I, OH, OA, OAc, SH, NO₂, NH₂, AcNH, HOOC und/oder AOOC substituierten Pyridyl-Z-, wobei 1-Pyridyl-Z ausgenommen ist, Oxo-dihydro-pyridyl-, Oxo-dihydro-pyridazinyl-oxy, Oxo-dihydro-pyridazinyl-thio, Oxo-dihydro-pyridazinyl-amino- oder Oxo-dihydro-pyrrolyl-rest, wobei die nicht über O, S oder NH an den Benzoxazinring gebundenen Reste auch vollständig oder partiell hydriert sein können,
R⁴ und R⁵ jeweils H, A, OH, AO, CHO, ACO, ACS, HOOC, AOOC, AO-CS, ACOO, A-CS-O, Hydroxyalkyl mit 1-6 C-Atomen, Mercaptoalkyl mit 1-6 C-Atomen, NO₂, NH₂, NHA, NA₂, CN, F, Cl, Br, I, CF₃, ASO, ASO₂, AO-SO, AO-SO₂, AcNH, AO-CO-NH, H₂NSO, HANSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkyl, Nitroalkyl, Cyanalkyl, A-C(=NOH) oder A-C(=NNH₂),
A Alkyl mit 1-6 C-Atomen,
-alkyl Alkylen mit 1-6 C-Atomen,
Ac Alkanoyl mit 1-8 C-Atomen oder Aroyl mit 7-11 C-Atomen und
Z O oder S
bedeuten,
worin jedoch R³ nur dann einen 2-Oxopyrrolidino- oder einen 2-Oxo-piperidino-Rest darstellt und gleichzeitig R⁴ H bedeutet, wenn R⁵ ungleich H, A, AO, F, Cl, Br oder I ist,
sowie deren Salze.

2. a) 2,2-Dimethyl-4-(2-oxo-1,2-dihydro-1-pyridyl)-2H-1,3-benzoxazin;
b) 2,2-Dimethyl-4-(2-oxo-1,2-dihydro-1-pyridyl)-6-cyan-2H-1,3-benzoxazin;
c) 2,2-Dimethyl-4-(2-pyridyl-oxy)-6-cyan-2H-1,3-benzoxazin;
d) 2,2-Dimethyl-4-(6-hydroxy-3-pyridazinyl-oxy)-6-cyan-2H-1,3-benzoxazin;
e) 2,2-Dimethyl-4-(2-oxo-1,2-dihydro-1-pyridyl)-6-brom-2H-1,3-benzoxazin.

3. Verfahren zur Herstellung von Benzoxazinderivaten der Formel I gemäß Anspruch 1, dadurch gekennzeichnet, daß man ein Benzoxazin der Formel II 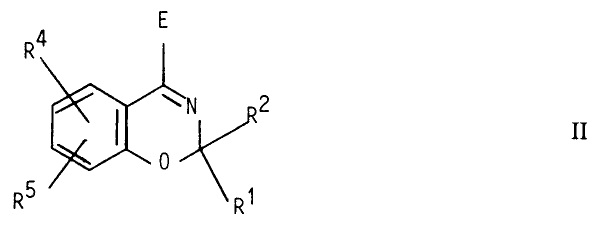 worin
E Cl, Br, I oder eine reaktionsfähig veresterte OH-Gruppe bedeutet und
R¹, R², R⁴ und R⁵ die bei Formel I angegebenen Bedeutungen haben,
mit einer Verbindung der Formel III
R³-H III
worin R³ die bei Formel I angegebene Bedeutung hat,
oder mit einem ihrer reaktionsfähigen K- oder Na-Salzen umsetzt und/oder daß man in einer Verbindung der Formel I einen oder mehrere der Reste R³, R⁴ und/oder R⁵ in andere Reste R³, R⁴ und/oder R⁵ umwandelt und/oder daß man eine basische Verbindung der Formel I durch Behandeln mit einer Säure in eines ihrer Säureadditionalsalze umwandelt.

4. Verfahren zur Herstellung pharmazeutischer Zübereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verbindungen der Formel I zur Bekämpfung von Krankheiten.

7. Verwendung von Verbindungen der Formel I zur Herstellung eines Arzneimittels.

8. Verwendung von Verbindungen der Formel I bei der Bekämpfung von Krankheiten.

## Patentansprüche (Patentansprüche für folgende(n) Vertragsstaat(en): ES)

1. Verfahren zur Herstellung von Benzoxazinderivaten der Formel I 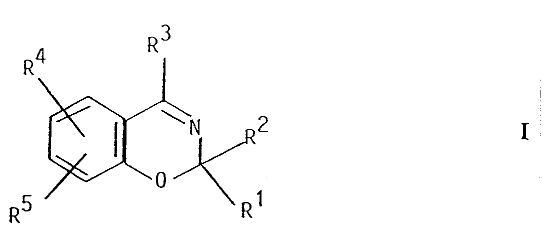 worin
R¹ und R² jeweils H oder A, oder zusammen auch Alkylen mit 3-6 C-Atomen,
R³ einen unsubstituierten oder ein- oder zweifach durch A, F, Cl, Br, I, OH, OA, OAc, SH, NO₂, NH2, AcNH, HOOC und/oder AOOC substituierten Pyridyl-Z-, wobei 1-Pyridyl-Z-ausgenommen ist, Oxo-dihydro-pyridyl, Oxy-dihydro-pyridazinyl-oxy, Oxo-dihydro-pyridazinyl-thio, Oxo-dihydro-pyridazinyl-amino oder Oxo-dihydro-pyrrolylrest, wobei die nicht über O, S oder NH an den Benzoxazinring gebundenen Reste auch vollständig oder partiell hydriert sein können,
R⁴ und R⁵ jeweils H, A, OH, OA, CHO, ACO, ACS, HOOC, AOOC, AO-CS, ACOO, A-CS-O, Hydroxyalkyl mit 1-6 C-Atomen, Mercaptoalkyl mit 1-6 C-Atomen, NO₂, NH₂, NHA, NA₂, CN, F, Cl, Br, I, CF₃, ASO, ASO₂, AO-SO, AO-SO₂, AcNH, AO-CO-NH, H₂NSO, HANSO, H₂NSO₂, HANSO₂, A₂ NSO₂, H₂NCO, HANCO, A₂NCO, H₂NCS, HANCS, A₂NCS, ASONH, ASO₂NH, AOSONH, AOSO₂NH, ACO-alkyl, Nitroalkyl, Cyanalkyl, A-C(=NOH) oder A-C(=NNH₂),
A Alkyl mit 1-6 C-Atomen und
-alkyl Alkylen mit 1-6 C-Atomen
Ac Alkanoyl mit 1-8 C-Atomen oder Aroyl mit 7-11 C-Atomen und
Z O oder S
bedeuten
worin jedoch R³ nur dann einen 2-Oxo-pyrrolidino- oder einen 2-Oxo-piperidinorest darstellt und gleichzeitig R⁴ H bedeutet, wenn R⁵ ungleich H, A, AO, F, Cl, Br oder I ist,
sowie deren Salze, dadurch gekennzeichnet, daß man ein Benzoxazinderivat der Formel II 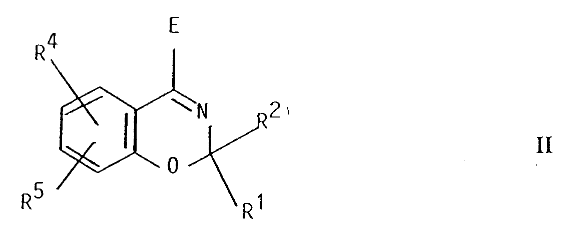 worin
E Cl, Br, I oder eine reaktionsfähig veresterte OH-Gruppe bedeutet und
R¹, R², R⁴ und R⁵ die bei Formel I angegebenen Bedeutungen haben,
mit einer Verbindung der Formel III
R³-H III
worin R³ die bei Formel I angegebene Bedeutung hat,
oder mit einem ihrer readionsfähigen K- oder Na-Salzen umsetzt und/oder daß man eine basische Verbindung der Formel I durch Behandeln mit einer Säure in eines ihrer Säureadditionssalze umwandelt.

2. Verfahren zur Herstellung von
a) 2,2-Dimethyl-4-(2-oxo-1,2-dihydro-1-pyridyl)-2H-1,3-benzoxazin;
b) 2,2-Dimethyl-4-(2-oxo-1,2-dihydro-1-pyridyl)-6-cyan-2H-1,3-benzoxazin;
c) 2,2-Dimethyl-4-(2-pyridyl-oxy)-6-cyan-2H-1,3-benzoxazin;
d) 2,2-Dimethyl-4-(6-hydroxy-3-pyridazinyl-oxy)-6-cyan-2H-1,3-benzoxazin;
e) 2,2-Diemthyl-4-(2-oxo-1,2-dihydro-1-pyridyl)-6-brom-2H-1,3-benzoxazin,
gemäß Anspruch 1.

3. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

## Claims (Claims for the following Contracting State(s): AT, BE, CH, DE, FR, GB, GR, IT, LI, LU, NL, SE)

1. Benoxazine derivatives of the formula I 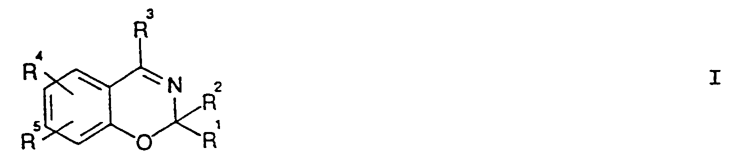 in which
R¹ and R² are each H or A, or, together, are alternatively alkylene having 3-6 C atoms,
R³ is a pyridyl-Z-, 1-pyridyl 1-Z being excepted, oxo-dihydro-pyridyl-, oxo-dihydro-pyridazinyl-oxy, oxo-dihydro-pyridazinyl-thio, oxo-dihydro-pyridazinyl-amino- or oxo-dihydro-pyrrolyl radical, each of which is unsubstituted or monosubstituted or disubstituted by A, F, Cl, Br, I, OH, OA, OAc, SH, NO₂, NH₂, AcNH, HOOC and/or AOOC, it being possible for the radicals which are not bonded to the benzoxazine ring via O, S or NH to also be completely or partially hydrogenated,
R⁴ and R⁵ are each H, A, OR, AO, CHO, ACO, ACS, HOOC, AOOC, AO-CS, ACOO, A-CS-O, hydroxyalkyl having 1-6 C atoms, mercaptoalkyl having 1-6 C atoms, NO₂, NH₂, NHA, NA₂, CN, F, Cl, Br, I, CF₃, ASO, ASO₂, AO-SO, AO-SO₂, AcNH, AO-CO-NH, H₂NSO, HANSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkyl, nitroalkyl, cyanoalkyl, A-C(=NOH) or A-C(=NNH₂),
A is alkyl having 1-6 C atoms,
-alkyl is alkylene having 1-6 C atoms,
Ac is alkanoyl having 1-8 C atoms or aroyl having 7-11 C atoms, and
Z is O or S,
but in which R³ is only a 2-oxopyrrolidino or 2-oxo-piperidino radical and R⁴ is simultaneously H if R⁵ is not H, A, AO, F, Cl, Br or I,
and salts thereof.

2. a) 2,2-Dimethyl-4-(2-oxo-1,2-dihydrol-1-pyridyl)-2H-1,3-benzoxazine;
b) 2,2-Dimethyl-4-(2-oxo-1,2-dihydro-1-pyridyl)-6-cyano-2H-1,3-benzoxazine;
c) 2,2-Dimethyl-4-(2-pyridyl-oxy)-6-cyano-2H-1,3-benzoxazine;
d) 2,2-Dimethyl-4-(6-hydroxy-3-pyridazinyloxy)-6-cyano-2H-1,3-benzoxazine;
e) 2,2-Dimethyl-4-(2-oxo-1,2-dihydro-1-pyridyl)-6-bromo-2H-1,3-benzoxazine.

3. Process for the preparation of benzoxazine derivatives of the formula I according to Claim 1, characterized in that a benzoxazine of the formula II 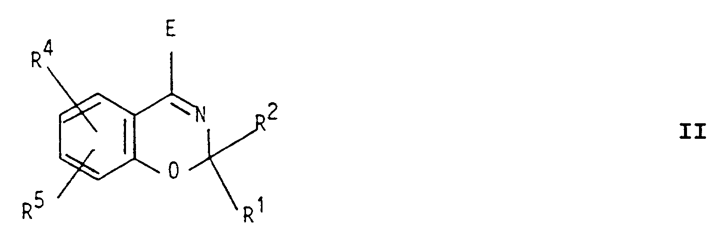 in which
E is Cl, Br, I or a reactively esterified OH group and
R¹, R², R⁴ and R⁵ are as defined in the case of formula I,
is reacted with a compound of the formula III
R³-H III
in which R³ is as defined in the case of formula I,
or with one of its reactive K or Na salts,
and/or in that, in a compound of the formula I, one or more of the radicals R³, R⁴ and/or R⁵ are converted into other radicals R³, R⁴ and/or R⁵, and/or in that a basic compound of the formula I is converted into one of its acid-addition salts by treatment with an acid.

4. Process for the preparation of pharmaceutical preparations, characterized in that a compound of the formula I and/or a physiologically acceptable salt thereof is converted into a suitable dosage form together with at least one solid, liquid or semi-liquid carrier or adjuvant and optionally in combination with one or more further active compound(s).

5. Pharmaceutical preparation, containing at least one compound of the formula I and/or a physiologically acceptable salt thereof.

6. Compounds of the formula I for combating diseases.

7. Use of compounds of the formula I for the preparation of a medicament.

8. Use of compounds of the formula I in combating diseases.

## Claims (Claims for the following Contracting State(s): ES)

1. Process for the preparation of benzoxazine derivatives of the formula I 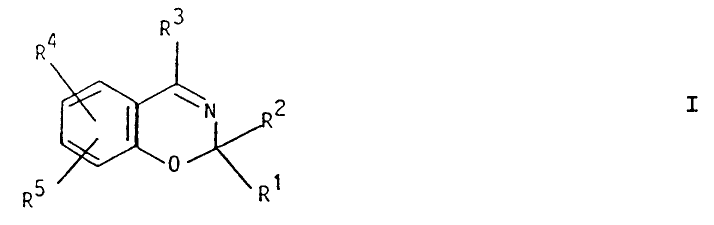 in which
R¹ and R² are each H or A, or, together, are alternatively alkylene having 3-6 C atoms,
R³ is a pyridyl-Z-, 1-pyridyl-Z- being excepted, oxo-dihydro-pyridyl-, oxo-dihydro-pyridazinyl-oxy, oxo-dihydro-pyridazinyl-thio, oxo-dihydro-pyridazinyl-amino- or oxo-dihydro-pyrrolyl radical, each of which is unsubstituted or monosubstituted or disubstituted by A, F, Cl, Br, I, OH, OA, OAc, SH, NO₂, NH₂, AcNH, HOOC and/or AOOC, it being possible for the radicals which are not bonded to the benzoxazine ring via O, S or NH to also be completely or partially hydrogenated,
R⁴ and R⁵ are each H, A, OH, OA, CHO, ACO, ACS, HOOC, AOOC, AO-CS, ACOO, A-CS-O, hydroxyalkyl having 1-6 C atoms, mercaptoalkyl having 1-6 C atoms, NO₂, NH₂, NHA, NA₂, CN, F, Cl, Br, I, CF₃, ASO, ASO₂, AO-SO, AO-SO₂, AcNH, AO-CO-NH, H₂NSO, HANSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkyl, nitroalkyl, cyanoalkyl, A-C(=NOH) or A-C(=NNH₂),
A is alkyl having 1-6 C atoms, and
-alkyl is alkylene having 1-6 C atoms,
Ac is alkanoyl having 1-8 C atoms or aroyl having 7-11 C atoms, and
Z is O or S,
but in which R³ is only a 2-oxopyrrolidino or 2-oxo-piperidino radical and R⁴ is simultaneously H if R⁵ is not H, A, AO, F, Cl, Br or I,
and salts thereof, characterized in that a benzoxazine derivative of the formula II 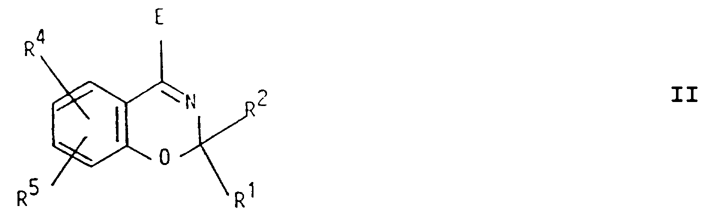 in which
E is Cl, Br, I or a reactively esterified OH group and
R¹, R², R⁴ and R⁵ are as defined in the case of formula I,
is reacted with a compound of the formula III
R³-H III
in which R³ is as defined in the case of formula I,
or with one of its reactive K or Na salts thereof,
and/or in that a basic compound of the formula I is converted into one of its acid-addition salts by treatment with an acid.

2. Process for the preparation of
a) 2,2-Dimethyl-4-(2-oxo-1,2-dihydro-1-pyridyl)-2H-1,3-benzoxazine;
b) 2,2-Dimethyl-4-(2-oxo-1,2-dihydro-1-pyridyl)-6-cyano-2H-1,3-benzoxazine;
c) 2,2-Dimethyl-4-(2-pyridyl-oxy)-6-cyano-2H-1, 3-benzoxazine;
d) 2,2-Dimethyl-4-(6-hydroxy-3-pyridazinyl-oxy)-6-cyano-2H-1,3-benzoxazine;
e) 2,2-Dimethyl-4-(2-oxo-1,2-dihydro-1-pyridyl)-6-bromo-2H-1,3-benzoxazine according to Claim 1.

3. Process for the preparation of pharmaceutical preparations, characterized in that a compound of the formula I and/or a physiologically acceptable salt thereof is converted into a suitable dosage form together with at least one solid, liquid or semi-liquid carrier or adjuvant and optionally in combination with one or more further active compound(s).

## Revendications (Revendications pour l'(les) Etat(s) contractant(s) suivant(s): AT, BE, CH, DE, FR, GB, GR, IT, LI, LU, NL SE)

1. Dérivés de benzoxazine de formule I 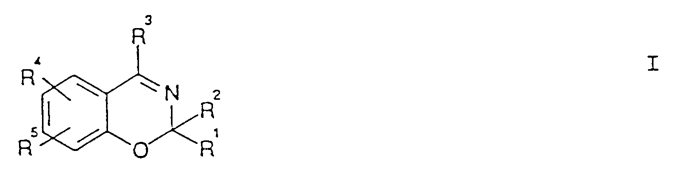 dans laquelle
R¹ et R² représentent respectivement H ou A, ou ensemble également un groupe alkylène contenant de 3 à 6 atomes de carbone;
R³ représente un radical pyridyle-Z-, le radical 1-pyridyle-Z- étant exclu, un radical oxo-dihydro-pyridyle, un radical oxo-dihydro-pyridazinyl-oxy, un radical oxo-dihydro-pyridazinyl-thio, un radical oxo-dihydro-pyridazinyl-amino ou un radical oxo-dihydro-pyrrolyle non substitués ou substitués une ou deux fois par A, F, CI, Br, I, OH, OA, OAc, SH, NO₂, NH₂, AcNH, HOOC et/ou AOOC, dans lequel les radicaux qui ne sont pas reliés au noyau benzoxazine à l'intervention de O, S ou NH peuvent également être complètement ou partiellement hydrogénés,
R⁴ et R⁵ représentent respectivement H, A, OH, AO, CHO, ACO, ACS, HOOC, AOOC, AO-CS, ACOO, A-CS-O, un groupe hydroxyalkyle contenant de 1 à 6 atomes de carbone, un groupe mercapto-alkyle contenant de 1 à 6 atomes de carbone, NO₂, NH₂, NHA, NA₂, CN, F, Cl, Br, I, CF₃, ASO, ASO₂, AO-SO, AO-SO₂, AcNH, AO-CO-NH, H₂NSO, HANSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, un groupe ACO-alkyle, un groupe nitro-alkyle, un groupe cyanalkyle, un groupe A-C(=NOH) ou un groupe A-C(=NNH₂),
A représente un groupe alkyle contenant de 1 à 6 atomes de carbone,
-alkyle représente un groupe alkylène contenant de 1 à 6 atomes de carbone,
Ac représente un groupe alcanoyle contenant de 1 à 8 atomes de carbone ou un groupe aroyle contenant de 7 à 11 atomes de carbone, et
Z représente O ou S,
dans laquelle, toutefois, R³ représente un radical 2-oxo-pyrrolidino ou un radical 2-oxo-pipéridino et R⁴ représente simultanément H, seulement lorsque R⁵ n'est pas égal à H, A, AO, F, Cl, Br ou I,
ainsi que leurs sels.

2. a) 2,2-diméthyl-4-(2-oxo-1,2-dihydro-1-pyridyl)-2H-1,3-benzoxazine
b) 2,2-diméthyl-4-(2-oxo-1,2-dihydro-1-pyridyl)-6-cyano-2H-1,3-benzoxazine
c) 2,2-diméthyl-4-(2-pyridyl-oxy)-6-cyano-2H-1,3-benzoxazine
d) 2,2-diméthyl-4-(6-hydroxy-3-pyridazinyl-oxy)-6-cyano-2H-1,3-benzoxazine
e) 2,2-diméthyl-4-(2-oxo-1,2-dihydro-1-pyridyl)-6-bromo-2H-1,3-benzoxazine

3. Procédé pour la préparation de dérivés de benzoxazine de formule I selon la revendication 1, caractérisé en ce qu'on fait réagir une benzoxazine de formule II 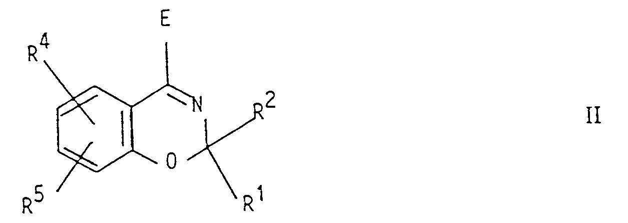 dans laquelle
E représente Cl, Br, I ou encore un groupe OH estérifié apte à réagir, et
R¹, R², R⁴ et R⁵ ont les significations indiquées dans la formule I
avec un composé de formule III
R³-H III
où R³ a la signification indiquée dans la formule I
ou bien avec un de ses sels de K ou de Na apte à réagir et/ou en ce qu'on transforme dans un composé de formule I un ou plusieurs des radicaux R³, R⁴ et/ou R⁵ en d'autres radicaux R³, R⁴ et/ou R⁵ et/ou en ce qu'on transforme un composé basique de formule I par traitement avec un acide en un de ses sels d'addition d'acides.

4. Procédé pour la formulation de préparations pharmaceutiques, caractérisé en ce qu'on amène à une forme posologique appropriée un composé de formule I et/ou un de ses sels physiologiquement acceptables conjointement avec au moins une substance de support ou auxiliaire solide, liquide ou semi-liquide et éventuellement en combinaison avec une ou plusieurs substances actives supplémentaires.

5. Préparation pharmaceutique caractérisée par une teneur en au moins un composé de formule I et/ou en un de ses sels physiologiquement acceptables.

6. Composés de formule I pour lutter contre des maladies.

7. Utilisation de composés de formule I pour la préparation d'un médicament.

8. Utilisation de composés de formule I pour lutter contre des maladies.

## Revendications (Revendications pour l'(les) Etat(s) contractant(s) suivant(s): ES)

1. Procédé pour la préparation de dérivés de benzoxazine de formule I 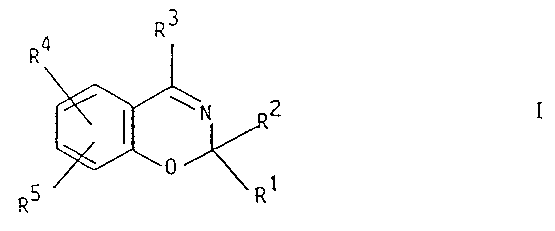 dans laquelle
R¹ et R² représentent respectivement H ou A, ou ensemble également un groupe alkylène contenant de 3 à 6 atomes de carbone;
R³ représente un radical pyridyle-Z-, le radical 1-pyridyle-Z- étant exclu, un radical oxo-dihydro-pyridyle, un radical oxo-dihydro-pyridazinyl-oxy, un radical oxo-dihydro-pyridazinyl-thio, un radical oxo-dihydro-pyridazinyl-amino ou un radical oxo-dihydro-pyrrolyle non substitués ou substitués une ou deux fois par A, F, Cl, Br, I, OH, OA, OAc, SH, NO₂, NH₂, AcNH, HOOC et/ou AOOC, dans lequel les radicaux qui ne sont pas reliés au noyau benzoxazine à l'intervention de O, S ou NH peuvent également être complètement ou partiellement hydrogénés,
R⁴ et R⁵ représentent respectivement H, A, OH, AO, CHO, ACO, ACS, HOOC, AOOC, AO-CS, ACOO, A-CS-O, un groupe hydroxyalkyle contenant de 1 à 6 atomes de carbone, un groupe mercaptoalkyle contenant de 1 à 6 atomes de carbone, NO₂, NH₂, NHA, NA₂, CN, F, Cl, Br, I, CF₃, ASO, ASO₂, AO-SO, AO-SO₂, AcNH, AO-CO-NH, H₂NSO, HANSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, un groupe ACO-alkyle, un groupe nitro-alkyle, un groupe cyanalkyle, un groupe A-C(=NOH) ou un groupe A-C(=NNH₂) ,
A représente un groupe alkyle contenant de 1 à 6 atomes de carbone,
-alkyle représente un groupe alkylène contenant de 1 à 6 atomes de carbone,
Ac représente un groupe alcanoyle contenant de 1 à 8 atomes de carbone ou un groupe aroyle contenant de 7 à 11 atomes de carbone, et
Z représente O ou S,
dans laquelle, toutefois, R³ représente un radical 2-oxo-pyrrolidino ou un radical 2-oxo-pipéridino et R⁴ représente simultanément H, seulement lorsque R⁵ n'est pas égal à H, A, AO, F, Cl, Br ou I,
et de leurs sels,
caractérisé en ce qu'on fait réagir un dérivé de benzoxazine de formule II 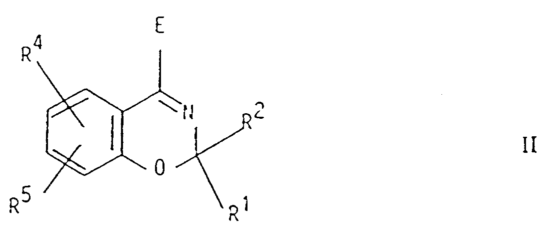 dans laquelle
E représente Cl, Br, I ou encore un groupe OH estérifié apte à réagir, et
R¹, R², R⁴ et R⁵ ont les significations indiquées dans la formule I
avec un composé de formule III
R³-H III
où R³ a la signification indiquée dans la formule I
ou bien avec un de ses sels de K ou de Na apte à réagir et/ou en ce qu'on transforme un composé basique de formule I par traitement avec un acide en un de ses sels d'addition d'acides.

2. Procédé pour la préparation de la
a) 2,2-diméthyl-4-(2-oxo-1,2-dihydro-1-pyridyl)-2H-1,3-benzoxazine
b) 2,2-diméthyl-4-(2-oxo-1,2-dihydro-1-pyridyl)-6-cyano-2H-1,3-benzoxazine
c) 2,2-diméthyl-4-(2-pyridyl-oxy)-6-cyano-2H-1,3-benzoxazine
d) 2,2-diméthyl-4-(6-hydroxy-3-pyridazinyl-oxy)-6-cyano-2H-1,3-benzoxazine
e) 2,2-diméthyl-4-(2-oxo-1,2-dihydro-1-pyridyl)-6-bromo-2H-1,3-benzoxazine
selon la revendication 1.

3. Procédé pour la formulation de préparations pharmaceutiques, caractérisé en ce qu'on amène à une forme posologique appropriée un composé de formule I et/ou un de ses sels physiologiquement acceptables conjointement avec au moins une substance de support ou auxiliaire solide, liquide ou semi-liquide et éventuellement en combinaison avec une ou plusieurs substances actives supplémentaires.
